# EUROPEAN PATENT SPECIFICATION

(11) **EP 2 535 078 B1**
(45) Date of publication and mention of the grant of the patent: **14.02.2018**
(21) Application number: 12171632.8
(22) Date of filing: 12.06.2012
(51) Int. Cl.: A61M 25/09

(54) **Guidewire**
Führungsdraht
Fil-guide

(30) Priority: 15.06.2011 JP 2011133051
(43) Date of publication of application: 19.12.2012
(73) Proprietor: ASAHI INTECC CO., LTD., Nagoya-shi, Aichi 463-0024 (JP)
(72) Inventor: Nagano, Satoshi, Nagoya-shi, Aichi 463-0024 (JP); Maki, Hideaki, Nagoya-shi, Aichi 463-0024 (JP); Kanazawa, Yuuya, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 0 815 894
- EP-A1- 1 468 707
- EP-A1- 2 263 737
- US-A1- 2005 228 431
- US-A1- 2009 112 127

## Description

### BACKGROUND OF THE INVENTION

### 1. Field of the Invention

The present invention relates to a guidewire.

### 2. Description of the Related Art

Guidewires are known as medical mechanical devices used to guide a device such as a balloon or a stent to a lesion in percutaneous transluminal coronary angioplasty (PTCA).

United States Patent No. 5,345,945 discloses an example of such a guidewire which includes a core shaft and a coil body. The core shaft includes a small-diameter front section and a large-diameter rear section. The coil body is wound around the outer periphery of the front section and includes a tapered section having a diameter that decreases toward a front end portion of the front section. The front section and the rear section of the core shaft correspond to a distal portion and a proximal portion, respectively, of the guidewire. The distal portion of the guidewire is inserted into the body, and the proximal portion of the guidewire is operated by an operator, such as a doctor.

Other known guidewire is disclosed in US 2009/112127 A1. US 2009/112127 A1 discloses a guidewire with a core shaft, an outer coil body covering a front section of the core shaft, and an inner coil body disposed between the core shaft.

Since the guidewire according to the related art described in United States Patent No. 5,345,945 includes the coil body including the tapered section that has a diameter that decreases toward the front end portion, the guidewire has high performance of penetration into a lesion.

However, when the guidewire is rotated, pushed, or pulled after being advanced into the lesion, the coil body is easily deformed around the tapered section. This tendency increases, in particular, when the diameter of the distal portion of the guidewire is reduced, that is, when the diameter of the coil body is reduced, to improve the performance of penetration into a lesion.

### SUMMARY OF THE INVENTION

The present invention has the object to provide a guidewire having high torque-transmitting performance and comprising an outer coil body with a tapered section, which coil body is not easily deformed when the guidewire is rotated, pushed, or pulled after being advanced into the lesion.

The inventors of the present invention have studied the cause of deformation of the coil body, and found out that it is because the shape of the tapered section gradually changes from a large-diameter area to a small-diameter area, and external force, such as torsion and bending force, more easily concentrates at the tapered section compared to cylindrical sections having a uniform diameter.

As a result of further studies conducted by the inventors based on the above findings, it has been found that the deformation can be prevented by reinforcing the tapered section by arranging another coil body in the tapered section which itself is easily deformed. Thus, the guidewire according to the present invention has been completed.

According to an aspect of the present invention as defined in claim 1, a guidewire includes a core shaft including a front section including a front end portion and a rear section including a rear end portion, an outer coil body that covers the front section, and an inner coil body that is disposed in the outer coil body and covers the front section. The outer coil body includes a cylindrical large-diameter section positioned near the rear end portion, a cylindrical small-diameter section positioned near the front end portion, and a tapered section that connects the large-diameter section to the small-diameter section with a connecting portion provided therebetween, the tapered section having a diameter that decreases from an end near the rear end portion to an end near the front end portion. Of the large-diameter section, the small-diameter section, the connecting portion, and the tapered section, the inner coil body is disposed at least in the tapered section.

Preferred embodiments thereof are subject-matter of dependent claims and are described below with reference to the drawings.

### BRIEF DESCRIPTION OF THE DRAWINGS

Fig. 1 is a schematic sectional view of a guidewire according to a first embodiment of the present invention taken along a longitudinal direction of the guidewire.
Fig. 2 is an enlarged sectional view of an area around a distal portion of the guidewire illustrated in Fig. 1.
Fig. 3 is an enlarged sectional view of an area around a distal portion of a guidewire according to a second embodiment of the present invention.
Fig. 4 is an enlarged sectional view of an area around a distal portion of a guidewire according to a third embodiment of the present invention.
Fig. 5 is an enlarged sectional view of an area around a distal portion of a guidewire according to a fourth embodiment of the present invention.

### DESCRIPTION OF THE PREFERRED EMBODIMENTS

### First Embodiment

A guidewire according to a first embodiment of the present invention will now be described below with reference to the drawings.

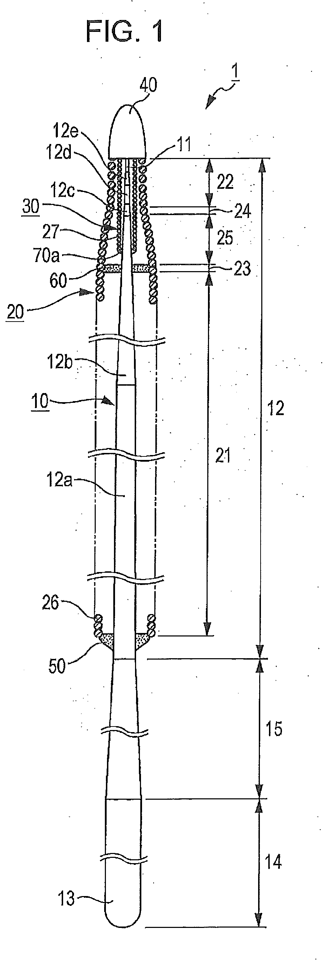

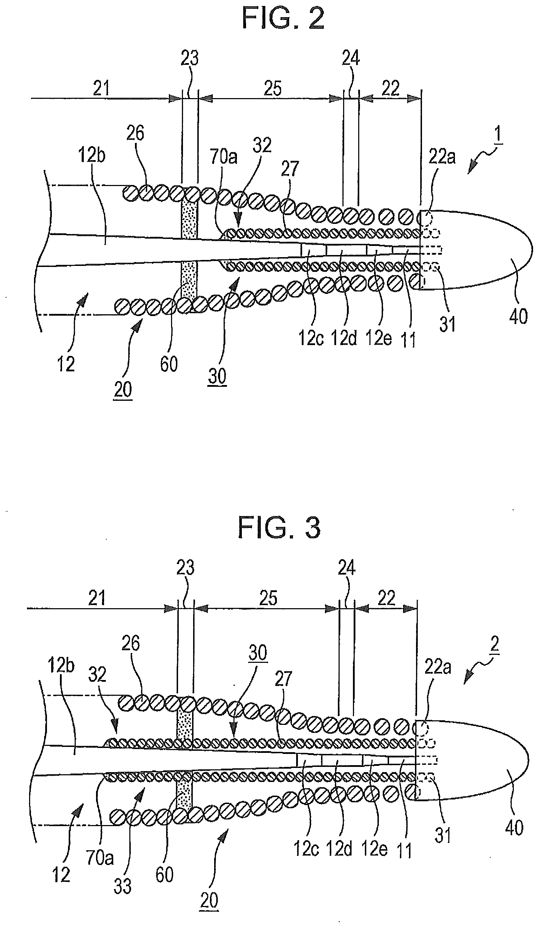

Fig. 1 is a schematic sectional view of a guidewire 1 according to a first embodiment of the present invention taken along a longitudinal direction of the guidewire 1. Fig. 2 is an enlarged sectional view of an area around a distal portion of the guidewire 1 illustrated in Fig. 1. In the following description, a distal portion of the guidewire 1 and a front section of a core shaft 10 are denoted by the same reference numeral 12, and a proximal portion of the guidewire 1 and a rear section of the core shaft 10 are denoted by the same reference numeral 14. In addition, a portion of an outer coil body 20 may be shown by broken lines, and detailed illustration of the portion of the outer coil body 20 may thus be omitted.

The guidewire 1 according to the present embodiment illustrated in Fig. 1 includes the core shaft 10, the outer coil body 20, and an inner coil body 30. The core shaft 10 includes the front section 12, which includes a front end portion 11, and the rear section 14, which includes a rear end portion 13. The outer coil body 20 covers the front section 12. The inner coil body 30 is disposed in the outer coil body 20 and covers the front section 12. The detailed structure of the guidewire 1 will now be described.

The core shaft 10 includes the front section 12, which includes the front end portion 11 and has a small diameter, a tapered intermediate section 15, which is connected to the front section 12, and the rear section 14, which is connected to the intermediate section 15, includes the rear end portion 13, and has a large diameter.

As illustrated in Figs. 1 and 2, the front section 12 includes, in order from the end near the rear end portion 13 to the front end portion 11, a first small-diameter portion 12a, a first tapered portion 12b, a second tapered portion 12c, a second small-diameter portion 12d, a third tapered portion 12e, and the front end portion 11, all of which are connected to each other. The small-diameter portions are columnar portions having a substantially uniform outer diameter, and the tapered portions are portions having a diameter that gradually decreases along the direction from the rear end portion 13 to the front end portion 11. As a whole, the front section 12 has a diameter that decreases stepwise from the end near the rear end portion 13 to the front end portion 11. Accordingly, the rigidity of the front section 12 gradually decreases and the flexibility thereof gradually increases toward the front end portion 11.

The outer coil body 20 is formed by helically winding a single wire 26, and has a tubular shape with a through hole therein. With regard to the detailed shape of the outer coil body 20, the outer coil body 20 includes a large-diameter section 21 having a uniform diameter and positioned at the rear end of the outer coil body 20, a tapered section 25 connected to the large-diameter section 21 with a first connecting portion 23 provided therebetween, and a small-diameter section 22 having a uniform diameter and positioned at the front end of the outer coil body 20 and connected to the tapered section 25 with a second connecting portion 24 provided therebetween. In this specification, any diameter of the large-diameter section 21, first connecting portion 23, tapered section 25, second connecting portion 24, and small-diameter section 22 of the outer coil body 20 corresponds to an outer diameter of the outer coil body 20.

The diameter of the tapered section 25 continuously decreases from the end near the large-diameter section 21 to the end near the small-diameter section 22. That is, the tapered section 25 has a truncated cone-shaped outer contour with a steadily decreasing diameter, i.e., a constant taper or cone angle, when referring to the longitudinal sectional view of the drawings. The external force, such as bending force, more easily concentrates at the tapered section 25 than at the large-diameter and small-diameter sections 21 and 22 having a uniform diameter, and therefore the tapered section 25 is easily deformed. In addition, the first and second connecting portions 23 and 24 have a shape, i.e., a diameter, that suddenly changes from the uniform diameter of the cylindrical large-diameter section 21 to a maximum diameter at the beginning of the tapered section 25 (first connecting portion 23), or from a minimum diameter of the tapered section 25 to the uniform diameter of the cylindrical small-diameter section 22. That is, each of the first connecting portion 23 and the second connecting portion 24 has, for example, a curved outer contour with a diameter which, unlike the diameter of the tapered section 25, does not decrease steadily but changes from uniform to decreasing (first connecting portion 23) and from decreasing to uniform (second connecting portion 24), respectively. In this specification, the first connecting portion 23 corresponds to several turns of wire in a longitudinal range along the longitudinal direction of the guidewire 1, in which the outer diameter of the coil body 20 decreases from that of the cylindrical large-diameter section 21, which has a substantially uniform diameter from the end near the rear end portion 13 to the end near the front end portion 11, to that of the beginning of the tapered section 25 so as to form a curved outer contour. Further, the second connecting portion 24 corresponds to several turns of wire in a longitudinal range along the longitudinal direction of the guidewire 1, in which the outer diameter decreases from that of the end of the tapered section 25, which has a diameter that decreases from the end near the rear end portion 13 to the end near the front end portion 11, to that of the cylindrical small-diameter section 22, which has a substantially uniform diameter so as to form a curved outer contour. Although a section corresponding to a single turn of wire is illustrated as each connecting portion in the drawings, this is because the wire that forms the outer coil body 20 is drawn as if it has a larger diameter than the actual diameter for simplification of the drawings, and the actual structure is different from those illustrated in the drawings. Therefore, the external force particularly easily concentrates at the first and second connecting portions 23 and 24 and the first and second connecting portions 23 and 24 are very easily deformed.

The front section 12 and the inner coil body 30 that partially covers the front section 12 are inserted in the outer coil body 20. The outer coil body 20 substantially entirely covers the front section 12 and the inner coil body 30.

In the small-diameter section 22, the wire 26 is loosely wound such that portions of the wire 26 that are adjacent to each other are spaced from each other. Therefore, the small-diameter section 22 is flexible. In the part of the outer coil body 20 other than the small-diameter section 22, the wire 26 is densely wound such that portions of the wire 26 that are adjacent to each other are in contact with each other. Therefore, this part of the outer coil body 20 is not easily twisted and is capable of efficiently transmitting a torque generated when the proximal portion 14 of the guidewire 1 is rotated to the front end portion of the distal portion 12 of the guidewire 1. The outer coil body 20 may instead be densely or loosely wound over the entire body thereof.

The inner coil body 30 is formed of a multiple-wire coil obtained by winding a plurality of wires 27. More specifically, the inner coil body 30 is formed by helically winding the wires 27, and has a tubular shape with a through hole therein. Therefore, plastic deformation of the inner coil body 30 does not easily occur compared to a single-wire coil obtained by winding a single wire. In addition, when a first end of the inner coil body 30 is rotated, a second end easily rotates so as to follow the first end. Thus, the inner coil body 30 has high rotation-following performance. The number of wires included in the inner coil body (multiple-wire coil) 30 is preferably 6 to 8.

Of the large-diameter section 21, the small-diameter section 22, the connecting portions 23 and 24, and the tapered section 25, the inner coil body 30 having the above-described structure is disposed at least in the tapered section 25. In the guidewire 1 illustrated in Fig. 1, the inner coil body 30 is disposed in the small-diameter section 22, the second connecting portion 24 that connects the small-diameter section 22 to the tapered section 25, and the tapered section 25.

However, as described in the following embodiments, the inner coil body 30 may instead be disposed so as to extend in the large-diameter section 21, the small-diameter section 22, the connecting portions 23 and 24, and the entire body of the tapered section 25.

A part of the front section 12, more specifically, a part of the first tapered portion 12b, the second tapered portion 12c, the second small-diameter portion 12d, the third tapered portion 12e, and the front end portion 11, is inserted in the inner coil body 30. Thus, the inner coil body 30 partially covers the front section 12.

The front end portion 11 of the core shaft 10, a front end portion 22a of the small-diameter section 22, and a front end portion 31 of the inner coil body 30 are fixed to each other by a front tip portion 40.

The front tip portion 40 has a conical shape, and the vertex of the cone serves as a front end portion of the guidewire 1. The front tip portion 40 is made of a solder material containing Au. The front tip portion 40 is preferably made of Sn-Au alloy.

A rear end portion of the first small-diameter portion 12a and a rear end portion of the large-diameter section 21 of the outer coil body 20 illustrated in Fig. 1 are fixed to each other by a rear-end solder part 50.

A front end portion of the large-diameter section 21 of the outer coil body 20 (the first connecting portion 23 that connects the large-diameter section 21 to the tapered section 25) and the first tapered portion 12b are fixed to each other by an intermediate solder part 60. The outer coil body 20 and the front section 12 may be additionally fixed to each other by one or more solder parts other than the rear-end solder part 50 and the intermediate solder part 60 at arbitrary positions.

A rear end portion 32 of the inner coil body 30 and the first tapered portion 12b are fixed to each other by an inner rear-end solder part 70a at a position closer to the front end portion 11 of the core shaft 10 than the intermediate solder part 60. The inner coil body 30 and the front section 12 (the first tapered portion 12b) may be additionally fixed to each other by one or more solder parts other than the inner rear-end solder part 70a at arbitrary positions.

### Manufacturing Method of Guidewire of First Embodiment

The guidewire according to the present embodiment may be manufactured by, for example, the following method. That is, first, the core shaft is produced by forming a core wire into the above-described shape by, for example, a taper cutting process or a pressing process. Then, the front section of the core shaft is inserted into the inner coil body and soldered to the inner coil body at a predetermined position. Then, the front section of the core shaft and the inner coil body are inserted into the outer coil body and soldered to the outer coil body at predetermined positions. Thus, the guidewire according to the present embodiment is manufactured.

The effects of the guidewire according to the present embodiment will now be described.
(1) In the guidewire according to the present embodiment, the inner coil body is disposed in the tapered section, which itself is easily deformed since the external force, such as bending force and torsion, easily concentrates thereat. Thus, the tapered section is reinforced. Accordingly, even when the guidewire is manually operated, the outer coil body including the tapered section is not easily deformed. As a result, the outer coil body is not easily damaged.
(2) The inner coil body is disposed in the tapered section, and a part of the guidewire including the tapered section has a double coil structure. Therefore, a torque generated when the proximal portion of the guidewire is rotated is efficiently transmitted to the front end portion of the distal portion of the guidewire through the outer coil body and the inner coil body. Thus, the guidewire according to the present embodiment has high torque-transmitting performance.
(3) The guidewire includes the outer coil body including the tapered section having a diameter that decreases from the end near the rear end portion of the core shaft to the end near the front end portion of the core shaft, and the distal portion of the guidewire is shaped such that the diameter thereof decreases toward the tip. Accordingly, the guidewire has high performance of penetration into a lesion.
(4) The external force concentrates particularly at the connecting portions that connect the tapered section to the cylindrical large-diameter and small-diameter sections since the connecting portions have a shape that suddenly changes. However, in the guidewire according to the present embodiment, the inner coil body is disposed not only in the tapered section but also in one of the connecting portions. Therefore, deformation of the outer coil body is further effectively prevented and the torque-transmitting performance can be improved.
(5) The small-diameter section is particularly flexible compared to the large-diameter section, and the strength of the small-diameter section may be low depending on the diameter thereof. However, in the guidewire according to the present embodiment, the inner coil body is disposed in the tapered section, the small-diameter section, and the second connecting portion that connects the tapered section to the small-diameter section. Therefore, deformation of the outer coil body can be further effectively prevented.
(6) The small-diameter section, which is located at the front end of the outer coil body, is originally flexible.
   In addition, since the small-diameter section is advanced to a deep part of the lesion, the external force easily concentrates at the small-diameter section. Therefore, the small-diameter section is particularly easily deformed. However, in the guidewire according to the present embodiment, since the inner coil body is disposed in the small-diameter section, deformation of the small-diameter section can be prevented.
(7) The front end portion of the core shaft, the front end portion of the small-diameter section of the outer coil body, and the front end portion of the inner coil body are fixed to each other by the front tip portion. Therefore, the torque applied to the rear section of the core shaft can be efficiently transmitted to the front tip portion through the outer coil body and the inner coil body. Thus, the torque-transmitting performance can be further improved.
(8) The inner coil body is formed of a multiple-wire coil obtained by winding a plurality of wires, and therefore the inner coil body is not easily plastically deformed and has high rotation-following performance. As a result, the above-described effects (1), (2), and (4) to (7) can be enhanced.
(9) Since the front tip portion has a conical shape,
   the performance of penetration into a lesion can be improved.
(10) The front tip portion is made of a solder material containing Au. Therefore, the front tip portion has a higher strength than that of a front tip portion made of, for example, a solder material containing Ag-Sn alloy, and does not easily break even when the length thereof is reduced.

### Second Embodiment

A guidewire according to a second embodiment of the present invention will now be described with reference to the drawings. The guidewire according to the present embodiment has a structure similar to that of the above-described guidewire according to the first embodiment except that the inner coil body is disposed in the large-diameter section, the first connecting portion, the tapered section, the second connecting portion, and the small-diameter section. Therefore, explanations of features similar to those of the guidewire according to the first embodiment will be omitted.

Fig. 3 is an enlarged sectional view of an area around a distal portion of a guidewire 2 according to the second embodiment of the present invention.

Referring to Fig. 3, in the guidewire 2 according to the present embodiment, a part of the first tapered portion 12b, the second tapered portion 12c, the second small-diameter portion 12d, the third tapered portion 12e, and the front end portion 11 are inserted in the inner coil body 30, and the inner coil body 30 is disposed in the large-diameter section 21, the first connecting portion 23, the tapered section 25, the second connecting portion 24, and the small-diameter section 22.

The front end portion of the large-diameter section 21 of the outer coil body 20 (the first connecting portion 23 that connects the large-diameter section 21 to the tapered section 25), an intermediate portion 33 of the inner coil body 30, and the first tapered portion 12b are fixed to each other by the intermediate solder part 60.

The rear end portion 32 of the inner coil body 30 and the first tapered portion 12b are fixed to each other by the inner rear-end solder part 70a at a position closer to the rear end portion 13 of the core shaft 10 than the intermediate solder part 60.

### Manufacturing Method of Guidewire of Second Embodiment

The guidewire according to the present embodiment is manufactured by an exemplary method similar to the manufacturing method of the guidewire according to the first embodiment except that a longer inner coil body is used.

The effects of the guidewire according to the present embodiment will now be described. The above-described effects (1) to (10) of the first embodiment can also be achieved by the guidewire according to the present embodiment. In addition, the following effects (11) and (12) can be additionally achieved.
(11) The external force concentrates particularly at the first and second connecting portions since the first and second connecting portions have a shape that suddenly changes. However, in the guidewire according to the present embodiment, the inner coil body is disposed not only in the tapered section but also in the first and second connecting portions. Therefore, deformation of the first and second connecting portions and the tapered section is further effectively prevented and the torque-transmitting performance can be further improved.
(12) The first connecting portion, the intermediate portion of the inner coil body, and the first tapered portion are fixed to each other by the intermediate solder part. Thus, the outer coil body and the inner coil body are more strongly fixed to each other. As a result, the torque-transmitting performance can be further improved.

### Third Embodiment

A guidewire according to a third embodiment of the present invention will now be described with reference to the drawings. The guidewire according to the present embodiment has a structure similar to that of the above-described guidewire according to the first embodiment except that the inner coil body is disposed in the first connecting portion, the tapered section, and the second connecting portion, but is not disposed in the large-diameter section or the small-diameter section. Therefore, explanations of features similar to those of the guidewire according to the first embodiment will be omitted.

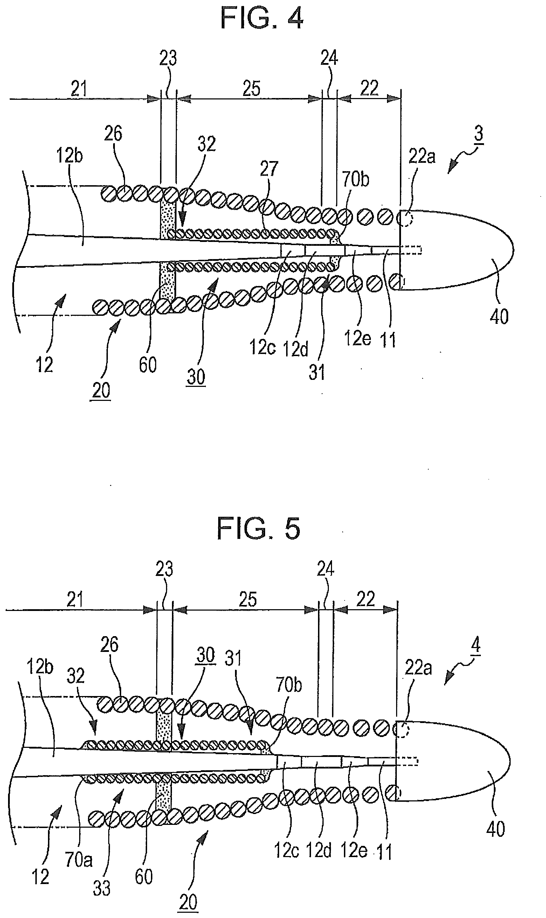

Fig. 4 is an enlarged sectional view of an area around a distal portion of a guidewire 3 according to the third embodiment of the present invention.

Referring to Fig. 4, in the guidewire 3 according to the present embodiment, a part of the first tapered portion 12b, the second tapered portion 12c, and a part of the second small-diameter portion 12d are inserted in the inner coil body 30, and the inner coil body 30 is disposed in the first connecting portion 23, the tapered section 25, and the second connecting portion 24.

The first connecting portion 23, the rear end portion 32 of the inner coil body 30, and the first tapered portion 12b are fixed to each other by the intermediate solder part (inner rear-end solder part) 60.

The front end portion 31 of the inner coil body 30 and the second small-diameter portion 12d are fixed to each other by an inner front-end solder part 70b.

### Manufacturing Method of Guidewire of Third Embodiment

The guidewire according to the present embodiment is manufactured by an exemplary method similar to the manufacturing method of the guidewire according to the first embodiment except that a shorter inner coil body is used and the soldering positions are changed.

The effects of the guidewire according to the present embodiment will now be described. The above-described effects (1) to (4) and (8) to (12) can also be achieved by the guidewire according to the present embodiment. In addition, the following effect (13) can be additionally achieved.
(13) Since the inner coil body is not disposed in the small-diameter section, the distal portion of the guidewire is particularly flexible in the area around the front end thereof (area corresponding to the small-diameter section). Therefore, a part of the guidewire in the area around the front end thereof can be easily bent at a predetermined angle (shaped) in advance. When the guidewire is shaped in advance, vascular selectivity for thin blood vessels can be increased and the guidewire can be precisely moved in the lesion. Thus, a guidewire having a high controllability can be provided.

### Fourth Embodiment

A guidewire according to a fourth embodiment of the present invention will now be described with reference to the drawings. The guidewire according to the present embodiment has a structure similar to that of the above-described guidewire according to the first embodiment except that the inner coil body is disposed in the large-diameter section, the first connecting portion, and the tapered section, but is not disposed in the second connecting portion or the small-diameter section. Therefore, explanations of features similar to those of the guidewire according to the first embodiment will be omitted.

Fig. 5 is an enlarged sectional view of an area around a distal portion of a guidewire 4 according to the fourth embodiment of the present invention.

Referring to Fig. 5, in the guidewire 4 according to the present embodiment, a part of the first tapered portion 12b is inserted in the inner coil body 30, and the inner coil body 30 is disposed in the large-diameter section 21, the first connecting portion 23, and the tapered section 25.

The rear end portion 32 of the inner coil body 30 and the first tapered portion 12b are fixed to each other by the inner rear-end solder part 70a.

The first connecting portion 23, the intermediate portion 33 of the inner coil body 30, and the first tapered portion 12b are fixed to each other by the intermediate solder part 60.

The front end portion 31 of the inner coil body 30 and the first tapered portion 12b are fixed to each other by the inner front-end solder part 70b.

### Manufacturing Method of Guidewire of Fourth Embodiment

The guidewire according to the present embodiment is manufactured by an exemplary method similar to the manufacturing method of the guidewire according to the first embodiment except that the soldering positions of the inner coil body are changed.

The effects of the guidewire according to the present embodiment will now be described. The above-described effects (1) to (4), (8) to (10), (12), and (13) can also be achieved by the guidewire according to the present embodiment. In addition, the following effect (14) can be additionally achieved.
(14) The rear end portion of the inner coil body is fixed to the first tapered portion of the core shaft, which is near the rear section thereof, by the inner rear-end solder part. Therefore, the torque applied to the rear section of the core shaft can be efficiently transmitted to the front end portion. Thus, the torque-transmitting performance can be further improved.

### Other Embodiments

In the guidewire according to the present invention, as described above, the small-diameter section is preferably positioned at the front end of the outer coil body. However, the small-diameter section may instead be disposed at a position shifted rearward from the front end of the outer coil body by a predetermined distance.

In the guidewire according to the present invention, as described above, the outer coil body may include a single set including the large-diameter section, the tapered section, and the small-diameter section. Alternatively, however, the outer coil body may include two or more sets which each include a large-diameter section, a tapered section, and a small-diameter section. In the case where the outer coil body includes two or more sets which each include a large-diameter section, a tapered section, and a small-diameter section, these sections are preferably arranged in such an order that the diameter of the outer coil body decreases stepwise from the end near the rear end portion of the core shaft to the end near the front end portion of the core shaft. For example, in the direction from the rear end portion to the front end portion of the core shaft, a large-diameter section, a tapered section, and a small-diameter section of a first set may be arranged in that order, and then a large-diameter section, a tapered section, and a small-diameter section of a second set may be arranged in that order. Preferably, the outer diameter of the small-diameter section in the first set is equal to or larger than the outer diameter of the large-diameter section in the second set. In the case where two or more sets which each include a large-diameter section, a tapered section, and a small-diameter section are provided, the dimension of the step between the large-diameter section and the small-diameter section can be reduced. Therefore, the guidewire can be more smoothly advanced into the lesion. In the case where two or more sets which each include a large-diameter section, a tapered section, and a small-diameter section are provided, the inner coil body may be disposed at least in the tapered section of one of the sets.

In the guidewire according to the present invention, the front tip portion preferably has a conical shape to improve the performance of penetration into a lesion. Alternatively, however, the front tip portion may have a hemispherical shape instead. In the case where the front tip portion has a hemispherical shape, even when the guidewire has high push-in performance based on high rigidity of the core shaft, the risk that the guidewire will penetrate a blood vessel can be reduced.

In the guidewire according to the present invention, the solder parts including the front-end solder part, the intermediate solder part, the rear-end solder part, the inner front-end solder part, and the inner rear-end solder part may be formed of, for example, aluminum alloy solder, silver solder, gold solder, zinc solder, Sn-Pb alloy solder, Sn-Au alloy solder, Pb-Ag alloy solder, or Sn-Ag alloy solder. Preferably, in particular, the solder parts are made of gold solder or Sn-Au alloy solder. In such a case, the strength of the solder parts can be increased.

In the guidewire according to the present invention, the core shaft may be made of, for example, a stainless steel, a superelastic alloy such as Ni-Ti alloy, a piano wire, or a tungsten wire. The stainless steel may be, for example, a martensitic stainless steel, a ferritic stainless steel, an austenitic stainless steel, an austenitic-ferritic duplex stainless steel, or a precipitation hardened stainless steel. Preferably, the core shaft is made of an austenitic stainless steel. In particular, SUS304, SUS316, or SUS316L is preferably used.

In the guidewire according to the present invention, the inner coil body is preferably formed of a multiple-wire coil obtained by winding a plurality of wires. However, the inner coil body may instead be formed of a single-wire coil obtained by winding a single wire. In the case where the inner coil body is formed of a single-wire coil, the inner coil body has a higher flexibility than that of the inner coil body formed of a multiple-wire coil.

In the guidewire according to the present invention, the inner coil body is formed of a single multiple-wire coil. However, the inner coil body may instead be formed by connecting a plurality of multiple-wire coils. In the case where the inner coil body is formed by connecting a plurality of multiple-wire coils, the multiple-wire coils may have different diameters (inner and outer diameters). In this case, the multiple-wire coils are preferably arranged such that the diameter thereof gradually decreases from the end near the rear end portion of the core shaft to the end near the front end portion of the core shaft. Accordingly, the flexibility gradually increases toward the front end portion of the guidewire, and the performance of penetration into a lesion can be improved while ensuring the flexibility of the distal portion of the guidewire.

In the guidewire according to the present invention, the wires that form the inner coil body may be made of, for example, a stainless steel, a superelastic alloy such as Ni-Ti alloy, a piano wire, or a tungsten wire. The stainless steel may be, for example, a martensitic stainless steel, a ferritic stainless steel, an austenitic stainless steel, an austenitic-ferritic duplex stainless steel, or a precipitation hardened stainless steel. Preferably, the wires are made of an austenitic stainless steel. In particular, SUS304, SUS316, or SUS316L is preferably used.

In the guidewire according to the present invention, the wire that forms the outer coil body may be made of, for example, a stainless steel, such as a martensitic stainless steel, a ferritic stainless steel, an austenitic stainless steel, an austenitic-ferritic duplex stainless steel, or a precipitation hardened stainless steel, a superelastic alloy such as Ni-Ti alloy, or a radiopaque metal, such as platinum, gold, or tungsten.

In the guidewire according to an example the distal portion of the guidewire may be shaped such that a bent portion that is bent at a predetermined angle is formed at a position separated from the front end portion of the front-end solder part toward the rear end portion of the core shaft by a predetermined distance. In the case where the guidewire is shaped, vascular selectivity can be increased and the guidewire can be precisely moved in the lesion. Thus, a guidewire having a high controllability can be provided. The shaping angle at which the bent portion is bent is preferably in the range of 10° to 45°, and the distance between the front-end solder part and the bent portion is preferably in the range of 0.5 mm to 5 mm. In this case, vascular selectivity for thin blood vessels and controllability in a lesion can be further increased.

The outer surface of the guidewire according to an example may be covered with a hydrophilic material. In such a case, the guidewire can be smoothly moved through a guiding catheter, a tube, or a body tissue by reducing the sliding friction.

Examples of the hydrophilic material include cellulose-based high-polymer materials, polyethylene-oxide-based high-polymer materials, maleic-anhydride-based high-polymer materials (e.g., maleic anhydride copolymers such as methyl vinyl ether-maleic anhydride copolymers), acrylamide-based high-polymer materials (e.g., polyacrylamides and polyglycidyl methacrylate-dimethylacrylamide (PGMA-DMAA) block copolymers), water-soluble nylons, polyvinyl alcohols, polyvinyl pyrrolidones, and hyaluronates. In particular, hyaluronates are preferably used.

In the guidewire according to an example a connector portion to which an extension guidewire can be attached may be provided at the rear end portion of the core shaft.

## Claims

1. A guidewire (1, 2, 3, 4) comprising:
a core shaft (10) including a front section (12) including a front end portion (11), and a rear section (14) including a rear end portion (13);
an outer coil body (20) that covers the front section (12); and
an inner coil body (30) that is disposed between the core shaft (10) and the outer coil body (20) and covers the front section (12),
wherein the outer coil body (20) includes
a cylindrical large-diameter section (21) positioned near the rear end portion (13),
a cylindrical small-diameter section (22) positioned near the front end portion (11), and
a tapered section (25) that connects the large-diameter section (21) to the small-diameter section (22) with a connecting portion provided therebetween, the tapered section (25) having a diameter that decreases from an end near the rear end portion (13) to an end near the front end portion (11), **characterized in that**
of the large-diameter section (21), the small-diameter section (22), the connecting portion, and the tapered section (25), the inner coil body (30) is disposed at least in the tapered section (25).

2. The guidewire according to Claim 1, wherein the connecting portion includes a first connecting portion (23) that connects the large-diameter section (21) to the tapered section (25) and a second connecting portion (24) that connects the small-diameter section (22) to the tapered section (25), and the inner coil body (30) is disposed at least in the tapered section (25) and at least one of the first and second connecting portions (23, 24).

3. The guidewire according to Claim 2, wherein the inner coil body (30) is disposed at least in the tapered section (25) and the first connecting portion (23).

4. The guidewire according to Claim 2, wherein the inner coil body (30) is disposed at least in the tapered section (25), the second connecting portion (24), and the small-diameter section (22).

5. The guidewire according to one of Claims 1 to 4, wherein
a front end portion of the large-diameter section (21), the inner coil body (30), and the core shaft (10) are fixed to each other by an intermediate solder part (60).

6. The guidewire according to one of Claims 1 to 5, wherein the front end portion (11) of the core shaft (10), a front end portion of the small-diameter section (22), and a front end portion of the inner coil body (30) are fixed to each other with a front tip portion (40).

7. The guidewire according to Claim 6, wherein the front tip portion (40) is formed of a solder material containing Au.

8. The guidewire according to one of Claims 1 to 7, wherein the inner coil body (30) is formed of a multiple-wire coil obtained by winding a plurality of wires.

## Patentansprüche

1. Führungsdraht (1, 2, 3, 4) mit:
einem Kernschaft (10), der einen vorderen Teil (12) mit einem vorderen Endabschnitt (11) und einen hinteren Teil (14) mit einem hinteren Endabschnitt (13) aufweist;
einem äußeren Wicklungskörper (20), der den vorderen Teil (12) umgibt; und
einem inneren Wicklungskörper (30), der zwischen dem Kernschaft (10) und dem äußeren Wicklungskörper (20) angeordnet ist und den vorderen Teil (12) umgibt,
wobei der äußere Wicklungskörper (20) aufweist:
einen zylindrischen, im Durchmesser großen Teil (21), der in der Nähe des hinteren Endabschnitts (13) liegt,
einen zylindrischen, im Durchmesser kleinen Teil (22), der in der Nähe des vorderen Endabschnitts (11) liegt, und
einen verjüngten Teil (25), der den im Durchmesser großen Teil (21) mit dem im Durchmesser kleinen Teil (22) über einen dazwischen liegenden Verbindungsabschnitt verbindet, wobei der verjüngte Teil (25) einen Durchmesser hat, der sich von einem Ende in der Nähe des hinteren Endabschnitts (13) zu einem Ende in der Nähe des vorderen Endabschnitts (11) hin verringert, **dadurch gekennzeichnet, dass**
was den im Durchmesser großen Teil (21), den im Durchmesser kleinen Teil (22), den Verbindungsabschnitt und den verjüngten Teil (25) betrifft, der innere Wicklungskörper (30) wenigstens in dem verjüngten Teil (25) angeordnet ist.

2. Führungsdraht nach Anspruch 1, wobei
der Verbindungsabschnitt einen ersten Verbindungsabschnitt (23), der den im Durchmesser großen Teil (21) mit dem verjüngten Teil (25) verbindet, und einen zweiten Verbindungsabschnitt (24), der den im Durchmesser kleinen Teil (22) mit dem verjüngten Teil (25) verbindet, aufweist, und
der innere Wicklungskörper (30) wenigstens in dem verjüngten Teil (25) und wenigstens in einem des ersten und zweiten Verbindungsabschnitts (23, 24) angeordnet ist.

3. Führungsdraht nach Anspruch 2, wobei der innere Wicklungskörper (30) wenigstens in dem verjüngten Teil (25) und in dem ersten Verbindungsabschnitt (23) angeordnet ist.

4. Führungsdraht nach Anspruch 2, wobei der innere Wicklungskörper (30) wenigstens in dem verjüngten Teil (25), in dem zweiten Verbindungsabschnitt (24) und in dem im Durchmesser kleinen Teil (22) angeordnet ist.

5. Führungsdraht nach einem der Ansprüche 1 bis 4, wobei
der vordere Endabschnitt des im Durchmesser großen Teils (21), der innere Wicklungskörper (30) und der Kernschaft (10) über ein Lot (60) aneinander befestigt sind.

6. Führungsdraht nach einem der Ansprüche 1 bis 5, wobei der vordere Endabschnitt (11) des Kernschafts (10), der vordere Endabschnitt des im Durchmesser kleinen Teils (22) und der vordere Endabschnitt des inneren Wicklungskörpers (30) über einen vorderen Spitzenabschnitt (40) aneinander befestigt sind.

7. Führungsdraht nach Anspruch 6, wobei der vordere Spitzenabschnitt (40) aus einem Lotmaterial gebildet ist, das Au enthält.

8. Führungsdraht nach einem der Ansprüche 1 bis 7, wobei der innere Wicklungskörper (30) aus einer durch Wickelung mehrere Drähte erhaltenen Mehrfachdrahtwicklung gebildet ist.

## Revendications

1. Fil-guide (1, 2, 3, 4) comprenant :
une broche d'enroulage (10) incluant une section avant (12) incluant une portion d'extrémité avant (11), et une section arrière (14) incluant une portion d'extrémité arrière (13) ;
un corps de bobine externe (20) qui couvre la section avant (12) ; et
un corps de bobine interne (30) qui est disposé entre la broche d'enroulage (10) et le corps de bobine externe (20) et couvre la section avant (12),
dans lequel le corps de bobine externe (20) inclut
une section cylindrique de grand diamètre (21) positionnée près de la portion d'extrémité arrière (13),
une section cylindrique de petit diamètre (22) positionnée près de la portion d'extrémité avant (11), et
une section effilée (25) qui raccorde la section de grand diamètre (21) à la section de petit diamètre (22) avec une portion de raccordement ménagée entre elles, la section effilée (25) ayant un diamètre qui diminue d'une extrémité près de la portion d'extrémité arrière (13) à une extrémité près de la portion d'extrémité avant (11), **caractérisé en ce que**
de la section de grand diamètre (21), la section de petit diamètre (22), la portion de raccordement, et la section effilée (25), le corps de bobine interne (30) est disposé au moins dans la section effilée (25).

2. Fil-guide selon la revendication 1, dans lequel la portion de raccordement inclut une première portion de raccordement (23) qui raccorde la section de grand diamètre (21) à la section effilée (25) et une seconde portion de raccordement (24) qui raccorde la section de petit diamètre (22) à la section effilée (25), et le corps de bobine interne (30) est disposé au moins dans la section effilée (25) et au moins l'une des première et seconde portions de raccordement (23, 24).

3. Fil-guide selon la revendication 2, dans lequel le corps de bobine interne (30) est disposé au moins dans la section effilée (25) et la première portion de raccordement (23).

4. Fil-guide selon la revendication 2, dans lequel le corps de bobine interne (30) est disposé au moins dans la section effilée (25), la seconde portion de raccordement (24), et la section de petit diamètre (22).

5. Fil-guide selon l'une des revendications 1 à 4, dans lequel
une portion d'extrémité avant de la section de grand diamètre (21), le corps de bobine interne (30), et la broche d'enroulage (10) sont fixés les uns aux autres par une partie de brasure tendre intermédiaire (60).

6. Fil-guide selon l'une des revendications 1 à 5, dans lequel la portion d'extrémité avant (11) de la broche d'enroulage (10), une portion d'extrémité avant de la section de petit diamètre (22), et une portion d'extrémité avant du corps de bobine interne (30) sont fixés les uns aux autres avec une portion d'embout avant (40).

7. Fil-guide selon la revendication 6, dans lequel la portion d'embout avant (40) est formée d'un matériau de brasure tendre contenant Au.

8. Fil-guide selon l'une des revendications 1 à 7, dans lequel le corps de bobine interne (30) est formé d'une bobine multi-fil obtenue par enroulement d'une pluralité de fils.
